# EUROPEAN PATENT SPECIFICATION

(11) **EP 3 654 988 B1**
(45) Date of publication and mention of the grant of the patent: **06.09.2023**
(21) Application number: 18779747.7
(22) Date of filing: 20.07.2018
(51) Int. Cl.: A61K 31/65, A61K 9/00, A61P 17/10, A61P 43/00

(54) **SARECYCLINE FOR THE TREATMENT OF NON-INFLAMMATORY ACNE LESIONS**
SARECYCLIN ZUR BEHANDLUNG VON NICHT-ENTZÜNDLICHEN AKNELÄSIONEN
SARECYCLINE DESTINÉE AU TRAITEMENT DES LÉSIONS ACNÉIQUES NON INFLAMMATOIRES

(30) Priority: 21.07.2017 US 201762535572 P
(43) Date of publication of application: 27.05.2020
(73) Proprietor: Almirall, LLC, Malvern, Pennsylvania 19355 (US)
(72) Inventor: BERK, David, Newport Beach CA 92660 (US); KAOUKHOV, Alexandre, Newport Beach CA 92660 (US); SNIUKIENE, Vilma, Lake Hopatcong NJ 07849 (US); SCHMITZ, Carsten, Irvine CA 92606 (US)
(74) Representative: J A Kemp LLP
(86) International application number: PCT/IB2018/000938
(87) International publication number: WO 2019/016609

(56) References cited:
- WO-A1-2012/155146
- WO-A1-2018/051102
- WO-A2-2008/079363
- Anonymous: "Double-Blind, Placebo-Controlled Study to Evaluate 3 Doses of P005672 in Treatment of Facial Acne Vulgaris - Full Text View - ClinicalTrials.gov", , 26 June 2012 (2012-06-26), XP055523788, Retrieved from the Internet: URL:https://clinicaltrials.gov/ct2/show/NC T01628549 [retrieved on 2018-11-14]
- J J LEYDEN ET AL: "Efficacy and Safety of Sarecycline, a Novel, Once-Daily, Narrow Spectrum Antibiotic for the Treatment of Moderate to Severe Facial Acne Vulgaris: Results of a Phase 2, Dose-Ranging Study.", JOURNAL OF DRUGS IN DERMATOLOGY, vol. 17, no. 3, 1 March 2018 (2018-03-01), pages 333-338, XP055523726, US ISSN: 1545-9616
- A MOORE ET AL: "Once-Daily Oral Sarecycline 1.5 mg/kg/day Is Effective for Moderate to Severe Acne Vulgaris: Results from Two Identically Designed, Phase 3, Randomized, Double-Blind Clinical Trials.", JOURNAL OF DRUGS IN DERMATOLOGY, vol. 17, no. 9, 1 September 2018 (2018-09-01), pages 987-996, XP055523735, US ISSN: 1545-9616
- A MOORE ET AL: "Once-Daily Oral Sarecycline 1.5 mg/kg/day Is Effective for Moderate to Severe Acne Vulgaris: Results from Two Identically Designed, Phase 3, Randomized, Double-Blind Clinical Trials.", JOURNAL OF DRUGS IN DERMATOLOGY, vol. 17, no. 9, 1 September 2018 (2018-09-01), pages 987-996, XP055523735, US ISSN: 1545-9616
- J J Leyden ET AL: "Efficacy and Safety of Sarecycline, a Novel, Once-Daily, Narrow Spectrum Antibiotic for the Treatment of Moderate to Severe Facial Acne Vulgaris: Results of a Phase 2, Dose-Ranging Study.", JOURNAL OF DRUGS IN DERMATOLOGY, vol. 17, no. 3, 1 March 2018 (2018-03-01), pages 333-338, XP055523726, US ISSN: 1545-9616

## Description

The present application claims the benefit of U.S. provisional application No. 62/535,572, filed July 21, 2017.

### FIELD OF THE INVENTION

The instant disclosure relates to a compound for use in the treatment of non-inflammatory acne vulgaris lesions.

### BACKGROUND OF THE INVENTION

Acne vulgaris, also referred to as acne, is typically an inflammatory skin disorder with inflammatory lesions such as papules and pustules. Acne may also be present with non-inflammatory lesions, also known as open and closed comedones. Acne is a disorder resulting from hormones affecting the sebaceous glands, which leads to plugged pores and outbreaks of lesions, or pimples. Acne is the most common skin disease in the United States, affecting nearly 17 million people. Severe acne can lead to disfiguration and permanent scarring.

Acne is described as a disorder of the pilosebaceous units (PSUs). Found over most of the body, PSUs consist of sebaceous glands, which make an oily substance that normally empties onto the skin surface through the opening of the follicle, also called a pore. When the follicle is plugged, the mixture of oil and cells allows bacteria that normally live on the skin to grow in the plugged follicles. Depending on the amount of oxygen that can penetrate the follicles, the bacteria may produce chemicals and enzymes and attract white blood cells that cause inflammation. The plugged follicle breaks down, the sebum, shed skin cells and bacteria disseminate into the nearby tissues, leading to lesions (i.e., inflammatory lesions) or pimples. Inflammatory lesions are characterized by the presence of papules, pustules, and nodules (cysts) and are classified as papulopustular and/or nodular. A severity grade based on a lesion count approximation is assigned as mild, moderate, or severe (Dermet Skin disease Atlas). Unlike inflammatory lesions, non-inflammatory lesions consist of open and closed comedones and usually appear before inflammatory lesions. Closed comedones or whiteheads appear as white microcysts. The follicular opening is barely perceptible. Open comedones or blackheads have a dilated follicular orifice that contains a plug with a dark surface. The opening may be small or very large.

Tetracycline compounds, or tetracyclines, are known "broad spectrum" antibiotics and have been widely used for therapeutic purposes. Tetracyclines are bacteriostatic drugs and act by binding reversibly to the 30S subunit of the bacterial ribosome. This inhibits the addition of amino acids to the growing peptide resulting in inhibition of protein synthesis. Tetracyclines also exhibit antiinflammatory and anti-collagenolytic properties which are not related to antibiotic activity (Cutis, 75(4 Suppl): 6-11, Apr. 2005).

For example, tetracyclines have been found to be highly effective pharmacologically against rickettsiae and several gram-positive and gram-negative bacteria, including lymphogranuloma venereum, inclusion conjunctivitis, and psittacosis. Examples of pharmaceutically active tetracycline analogue compositions may be found in U.S. Patent Nos. 2,980,584; 2,990,331; 3,062,717; 3,165,531; 3,454,697; 3,557,280; 3,674,859; 3,957,980; 4,018,889; 4,024,272; and 4,126,680. Some tetracyclines may also be used to treat inflammatory skin disorders, including dermatitis, psoriasis, pyoderma gangrenosum, acne, and rosacea.

After the widespread use of tetracyclines for both major and minor illnesses and diseases led to resistance to these antibiotics, substituted tetracycline compounds were developed to treat bacterial infections, inflammation, neoplasms, and other conditions. Examples of these tetracycline compounds include without limitation: chlortetracycline, doxycycline, minocycline, oxytetracycline, demeclocycline, methacycline, sancycline, chelocardin, rolitetracycline, lymecycline, apicycline, clomocycline, guamecycline, meglucycline, mepylcycline, penimepicycline, pipacycline, etamocycline, and penimocycline. For example, substituted tetracycline compounds have been disclosed in WO 2008/079339 and WO 2008/079363. The tetracycline compound sarecycline was investigated for its efficacy in the treatment of facial acne vulgaris (primarily inflammatory lesions, but also non-inflammatory lesions as a secondary measure) in https://clinicaltrials.gov/ct2/show/study/NCT01628549.

There remains a need for treatments of acne vulgaris, in particular, treatment of non-inflammatory lesions and symptoms thereof.

### SUMMARY OF THE INVENTION

The present invention relates to a compound, which compound is (4S,4aS,5aR,12aS)-4-dimethylamino-3, 10, 12, 12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt thereof, for use in a therapeutically effective amount in the treatment of non-inflammatory acne vulgaris lesion. One pharmaceutically acceptable salt may be a crystalline salt of (4S,4aS,5aR,12aS)-4-(dimethylamino)-3,10,12,12a-tetrahydroxy-7-[(methoxy(methyl)amino)methyl]-1,11-dioxo-1,4,4a,5,5a,6,11,12a-octahydro-naphthacene-2-carboxylic acid amide hydrochloride. This crystalline salt may be, for example, a *mono* hydrochloride, a *mono* mesylate, or a *mono* sulfate. Specifically, the crystalline salt may be (4S,4aS,5aR,12aS)-4-(dimethylamino)-3,10,12,12a-tetrahydroxy-7-[(methoxy(methyl)amino)methyl]-1,11-dioxo-1,4,4a,5,5a,6,11,12a-octahydro- naphthacene-2-carboxamide hydrochloride.

### BRIEF DESCRIPTION OF THE DRAWINGS

Fig. 1 shows the mean absolute reduction in non-inflammatory lesions for subjects having 30 or more non-inflammatory lesions at baseline as described in Example 1.
Fig. 2 shows the mean percent reduction in non-inflammatory lesions for subjects having 30 or more non-inflammatory lesions at baseline as described in Example 1.
Fig. 3 shows the mean absolute reduction in non-inflammatory lesions for subjects having 30 or more non-inflammatory lesions at baseline as described in Example 2.
Fig. 4 shows the mean percent reduction in non-inflammatory lesions for subjects having 30 or more non-inflammatory lesions at baseline as described in Example 2.
Fig. 5 shows the mean absolute reduction and the percent change in non-inflammatory lesions from baseline to final visit (week 12) as described in the Reference Example.

### DETAILED DESCRIPTION OF THE INVENTION

It has now been found that non-inflammatory lesions and/or symptoms thereof, particularly those associated with acne vulgaris or facial acne vulgaris, can be treated using (4S,4aS,5aR, 12aS)-4-dimethylamino-3, 10, 12, 12a-tetrahydroxy-7-[(methoxy(methyl)amino)-methyl]-1,11-dioxo-1,4,4a,5,5a,6,11,12a-octahydro-naphthacene-2-carboxylic acid amide (sarecycline) or a pharmaceutically acceptable salt thereof. It has also been found that prophylactic treatment for non-inflammatory lesions and/or symptoms thereof can be performed using (4S,4aS,5aR,12aS)-4-dimethylamino-3, 10, 12, 12a-tetrahydroxy-7-[(methoxy(methyl)amino)-methyl]-1,11-dioxo-1,4,4a,5,5a,6,11,12a-octahydro-naphthacene-2-carboxylic acid amide (sarecycline) or a pharmaceutically acceptable salt thereof.

Non-inflammatory lesions comprise open and closed comedones. An open comedo, commonly referred to as a blackhead, is a non-infected plugged hair follicle with a dilated or open orifice. A closed comedo, commonly referred to as a whitehead, is a non-infected plugged hair follicle having dilated follicular orifice that contains a plug with a dark skin surface.

Symptoms or signs of non-inflammatory lesions vary depending on the severity of the condition of the subject and may be associated with small epidermal cyst-like masses that manifest on the surface of the skin that may have a flesh-like color and/or dark spots, which may appear to be a brown or black color on the surface of the skin.

A therapeutically effective amount of the drug, or a safe and clinically effective amount of the drug, is administered , and, preferably, such administration is performed for a time period sufficient to reduce the number, size and/or occurrence of non-inflammatory lesions.

The term "therapeutically effective amount," as used herein, refers to an amount that may be effective to elicit the desired biological or medical response, including the amount of a compound that, when administered to a subject for treating a condition, is sufficient to affect such treatment for the condition. The effective amount will vary depending on the condition and its severity, as well as the age, weight and physical condition of the subject, as well as the duration of treatment.

The term "safe and clinically effective amount," as used herein, refers to an amount of a compound or composition high enough to significantly positively modify the symptoms and/or condition to be treated, but low enough to avoid serious side effects (at a reasonable risk/benefit ratio), within the scope of sound medical judgment. The safe and clinically effective amount of active ingredient for use in the method of the invention herein will vary with the particular condition being treated, the age and physical condition of the patient to be treated, the severity of the condition, the duration of the treatment, the nature of concurrent therapy, the particular active ingredient being employed, the particular pharmaceutically-acceptable excipients utilized, and like factors within the knowledge and expertise of the attending physician.

Subjects who may be treated, according to the present invention, are subjects who have and/or exhibit non-inflammatory lesions and subjects who may be prone to developing non-inflammatory lesions. In certain embodiments, the subject may have at least 10 non-inflammatory lesions, at least 20 non-inflammatory lesions, at least 30 non-inflammatory lesions, at least 40 non-inflammatory lesions, at least 50 non-inflammatory lesions, at least 60 non-inflammatory lesions, at least 70 non-inflammatory lesions, at least 80 non-inflammatory lesions, at least 90 non-inflammatory lesions, at least 100 non-inflammatory lesions, or at least 110 non-inflammatory lesions. In some embodiments, the subject may have fewer than 10 non-inflammatory lesions.

In one specific aspect, the subject in accordance with the present invention is a human. In another aspect, the subject is a non-human mammal.

The terms "treatment" or "treating," as used herein, include therapeutic and/or prophylactic treatment as described herein. With respect to the treatment of non-inflammatory lesions, such treatment includes the diminishment in the appearance, or size, or number of these non-inflammatory lesions on the skin and/or alleviation of at least one of the symptoms associated with non-inflammatory lesions.

The term "prophylactic treatment", as used herein, includes treatment that reduces the increase in the size and/or number of non-inflammatory lesions on the skin that would otherwise occur without such treatment. Preferably, the prophylactic treatment is a preventative treatment of a subject, wherein the preventative treatment may prevent an increase in the number of non-inflammatory lesions. The subject in need of the prophylactic treatment may have no non-inflammatory lesions or may have any number of non-inflammatory lesions, such as those mentioned above. For example, prophylactic treatment may be appropriate for a subject who is prone to developing non-inflammatory lesions but who may not exhibit non-inflammatory lesions at time of treatment.

The term "pharmaceutically acceptable salt," as used herein, refers to any adduct between two or more chemical species that are capable of undergoing proton transfer. As such, the term "pharmaceutically acceptable salt" encompasses adducts in which complete proton transfer has occurred, adducts in which partial proton transfer has occurred (e.g., in which an equilibrium mixture of charged and uncharged species is formed), and/or adducts in which proton transfer has not occurred but the chemical species are associated, e.g., by hydrogen bonding. It is understood that the term "pharmaceutically acceptable salt" also encompasses adducts in which close ion pairs are present. It will also be understood that the term "pharmaceutically acceptable salt" encompasses a continuum of adducts between those adducts in which complete proton transfer has occurred to form discrete ions and/or adducts in which two species are associated but proton transfer has not occurred or has only partially occurred. *See, e.g.,* Childs et al. Mol. Pharmaceutics, 2007, 4 (3), pp 323-338. A given pharmaceutically acceptable salt can contain one or multiple adducts on this continuum.

Pharmaceutically acceptable salts include salts of acidic or basic groups. Pharmaceutically acceptable acid addition salts include, but are not limited to, hydrochloride, hydrobromide, hydroiodide, nitrate, mesylate, sulfate, bisulfate, phosphate, acid phosphate, isonicotinate, acetate, lactate, salicylate, citrate, tartrate, pantothenate, bitartrate, ascorbate, succinate, maleate gentisinate, fumarate, gluconate, glucaronate, saccharate, formate, benzoate, glutamate, methanesulfonate, ethanesulfonate, benzensulfonate, ptoluenesulfonate and pamoate (i.e., 1,1'-methylene-bis-(2-hydroxy-3-naphthoate)) salts. Suitable base salts include, but are not limited to, aluminum, calcium, lithium, magnesium, potassium, sodium, zinc, and diethanolamine salts.

In some embodiments, the pharmaceutically acceptable salt may be a crystalline salt of (4S,4aS,5aR,12aS)-4-dimethylamino-3,10,12,12a-tetrahydroxy-7-[(methoxy(methyl)amino)-methyl]-1,11-dioxo-1,4,4a,5,5a,6,11,12a-octahydro-naphthacene-2-carboxylic acid amide. Such crystalline salt may be selected from the group consisting of *mono* hydrochloride, *mono* mesylate, and *mono* sulfate. These crystalline salts are described in U.S. Patent No. 9,255,068.

(4S,4aS,5aR,12aS)-4-(dimethylamino)-3,10,12,12a-tetrahydroxy-7-[(methoxy(methyl)amino)methyl]-1,11-dioxo-1,4,4a, 5, 5 a, 6,11,12a-octahydro-naphthacene-2-carboxamide hydrochloride has the structure represented by the following Formula I:

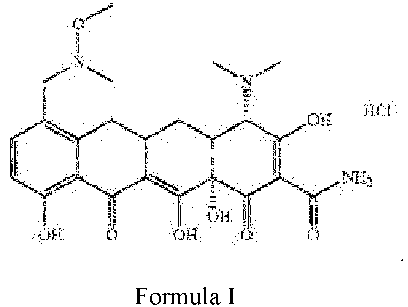

The therapeutically effective amount of (4S,4aS,5aR,12aS)-4-dimethylamino-3,10,12,12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt thereof will typically range from 0.75 mg/kg per day to 3.0 mg/kg per day. This amount may be from each of 0.75 mg/kg and 1.1 mg/kg to each of 1.5 mg/kg per day and 1.8 mg/kg per day. For example, the amount may be 3.0 mg/kg per day or 1.5 mg/kg per day.

In one embodiment, the therapeutically effective amount of (4S,4aS,5aR,12aS)-4-dimethylamino-3, 10, 12, 12a-tetrahydroxy-7-[(methoxy(methyl)amino)-methyl]-1,11-dioxo-1,4,4a, 5, 5 a,6,11,12a-octahydro-naphthacene-2-carboxylic acid amide or a pharmaceutically acceptable salt thereof employed is from 1 mg to 200 mg. This amount may be, for example, from each of 60 mg and 65 mg to each of 100 mg, 105 mg, 150 mg, 155 mg, and 160 mg. Individual doses of (4S,4aS,5aR,12aS)-4-dimethylamino-3, 10,12, 12a-tetrahydroxy-7-[(methoxy(methyl)amino)-methyl]-1,11-dioxo-1,4,4a,5,5a,6,11,12a-octahydro-naphthacene-2-carboxylic acid amide or its pharmaceutically acceptable salt may be administered in a dose amount (via a single or multiple dosage forms) of 60 mg, 65 mg, 100 mg, 105 mg, 150 mg, 155 mg, and 160 mg.

In certain embodiments, (4S,4aS,5aR,12aS)-4-dimethylamino-3, 10,12, 12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt thereof, in its therapeutically effective amount, is administered at least once weekly, bi-weekly, or daily. In certain embodiments, such administration is performed for at least 1 week, at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, or at least 12 weeks.

(4S,4aS,5aR, 12aS)-4-dimethylamino-3, 10, 12, 12a-tetrahydroxy-7-[(methoxy(methyl)amino)-methyl]-1,11-dioxo-1,4,4a, 5, 5 a,6,11,12a-octahydro-naphthacene-2-carboxylic acid amide or a pharmaceutically acceptable salt thereof may be administered to the subject in need thereof at approximately the same time each day. For example, administration may occur in the evening. Administration may take place at least 1 hour prior to, or 2 hours after, a meal, such as an evening meal.

Preferably, (4S,4aS,5aR,12aS)-4-dimethylamino-3,10,12,12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt thereof as discussed throughout this disclosure is administered to the subject orally via an oral dosage form.

The term "oral dose" or "oral dosage form," as used herein, refers to any pharmaceutical composition intended to be administered to the gastrointestinal tract of a subject via the mouth of said subject, and for purposes of the present disclosure, the oral dosage form as administered and/or delivered can be in liquid, solid, semi-solid, gelatinous form.

For instance, a solid oral dosage form may be a tablet, capsule, granule. Such oral dosage forms may contain various excipients such as microcrystalline cellulose, sodium citrate, calcium carbonate, dicalcium phosphate and glycine, along with various disintegrants such as starch (and preferably corn, potato or tapioca starch), alginic acid and certain complex silicates, together with granulation binders like polyvinylpyrrolidone, sucrose, gelatin and acacia. In a certain embodiment, the oral dosage form may be film coated. Additionally, lubricating agents such as magnesium stearate, sodium lauryl sulfate and talc may be used. Other solid compositions may also be employed as fillers in the oral dosage form including lactose or milk sugar, as well as high molecular weight polyethylene glycols.

When aqueous suspensions and/or elixirs are desired for oral administration, they may include various sweetening or flavoring agents, coloring matter or dyes, and, if so desired, emulsifying and/or suspending agents, together with such diluents as water, ethanol, propylene glycol, glycerin and various like combinations thereof.

In one embodiment, administering a therapeutically effective amount of (4S,4aS,5aR,12aS)-4-dimethylamino-3, 10, 12, 12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt may result in the mean percent reduction in non-inflammatory lesions from 2% to 75%. In another embodiment, such administration may result in the mean percent reduction in non-inflammatory lesions from 4% to 53%. In yet another embodiment, such administration may result in the mean percent reduction in non-inflammatory lesions from 14% to 43%. In another embodiment, such administration can result in the mean percent reduction in non-inflammatory lesions from 16% to 41%. In another embodiment, such administration can result in the mean percent reduction in non-inflammatory lesions from 17% to 39%. In another embodiment, such administration can result in the mean percent reduction in non-inflammatory lesions from 20% to 39%. In some embodiments, mean percent reduction of non-inflammatory lesions after 3 weeks of treatment with a therapeutically effective amount of (4S,4aS,5aR,12aS)-4-dimethylamino-3,10,12,12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt may range from 10% to 25%. In some embodiments, mean percent reduction of non-inflammatory lesions after 3 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt can range from 16% to 23%. In some embodiments, mean percent reduction of non-inflammatory lesions after 6 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt may range from 20% to 35%. In some embodiments, mean percent reduction of non-inflammatory lesions after 6 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt can range from 25% to 31%. In some embodiments, mean percent reduction of non-inflammatory lesions after 9 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt may range from 30% to 45%. In some embodiments, mean percent reduction of non-inflammatory lesions after 9 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt can range from 32% to 38%. In some embodiments, mean percent reduction of non-inflammatory lesions after 12 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt may range from 35% to 50%. In some embodiments, mean percent reduction of non-inflammatory lesions after 12 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt can range from 36% to 41%.

The difference in mean percent reduction in non-inflammatory lesions of a subject who receives a therapeutically effective amount of (4S,4aS,5aR,12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt compared to a placebo may range from 1% to 40%. In another embodiment, such administration may result in a difference in mean percent reduction in non-inflammatory lesions from 1% to 21%. In yet another embodiment, such administration may result in a difference in mean percent reduction in non-inflammatory lesions from 1% to 19%. In yet another embodiment, such administration can result in a difference in mean percent reduction in non-inflammatory lesions from 1% to 16%. In some embodiments, the difference in mean percent reduction of non-inflammatory lesions after 3 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt may range from 1% to 14%. In some embodiments, the difference in mean percent reduction of non-inflammatory lesions after 3 weeks of treatment with a therapeutically effective amount of (4S,4aS,5aR,12aS)-4-dimethylamino-3, 10, 12, 12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt may range from 1% to 10%. In some embodiments, the difference in mean percent reduction of non-inflammatory lesions after 3 weeks of treatment with a therapeutically effective amount of (4S,4aS,5aR,12aS)-4-dimethylamino-3,10,12,12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt can range from 1% to 8%. In some embodiments, the difference in mean percent reduction of non-inflammatory lesions after 6 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt may range from 1% to 17%.. In some embodiments, the difference in mean percent reduction of non-inflammatory lesions after 6 weeks of treatment with a therapeutically effective amount of (4S,4aS,5aR,12aS)-4-dimethylamino-3, 10, 12, 12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt may range from 1% to 12%. In some embodiments, the difference in mean percent reduction of non-inflammatory lesions after 6 weeks of treatment with a therapeutically effective amount of (4S,4aS,5aR,12aS)-4-dimethylamino-3,10,12,12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt can range from 7% to 11%. In some embodiments, the difference in mean percent reduction of non-inflammatory lesions after 9 weeks of treatment with a therapeutically effective amount of (4S,4aS,5aR,12aS)-4-dimethylamino-3, 10, 12, 12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt may range from 1% to 19%. In some embodiments, the difference in mean percent reduction of non-inflammatory lesions after 9 weeks of treatment with a therapeutically effective amount of (4S,4aS,5aR,12aS)-4-dimethylamino-3,10,12,12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt can range from 1% to 13%. In some embodiments, the difference in mean percent reduction of non-inflammatory lesions after 9 weeks of treatment with a therapeutically effective amount of (4S,4aS,5aR,12aS)-4-dimethylamino-3, 10, 12, 12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt can range from 6% to 10%. In some embodiments, the difference in mean percent reduction of non-inflammatory lesions after 12 weeks of treatment with a therapeutically effective amount of (4S,4aS,5aR,12aS)-4-dimethylamino-3,10,12,12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt may range from 1% to 20%. In some embodiments, the difference in mean percent reduction of non-inflammatory lesions after 12 weeks of treatment with a therapeutically effective amount of (4S,4aS,5aR,12aS)-4-dimethylamino-3, 10, 12, 12a-tetrahydroxy-7-[(methoxy(methyl)amino)-methyl]-1,11-dioxo-1,4,4a, 5, 5 a,6,11,12a-octahydro-naphthacene-2-carboxylic acid amide or a pharmaceutically acceptable salt can range from 1% to 15%. In some embodiments, the difference in mean percent reduction of non-inflammatory lesions after 12 weeks of treatment with a therapeutically effective amount of (4S,4aS,5aR,12aS)-4-dimethylamino-3,10,12,12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt can range from 5% to 11%..

In one embodiment, administering a therapeutically effective amount of (4S,4aS,5aR,12aS)-4-dimethylamino-3, 10, 12, 12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt may result in a mean absolute reduction in non-inflammatory lesions from 1 lesion to 40 lesions. In another embodiment, such administration may result in the mean absolute reduction in non-inflammatory lesions from 1 lesions to 29 lesions. In another embodiment, such administration may result in the mean absolute reduction in non-inflammatory lesions from 5 lesions to 23 lesions. In another embodiment, such administration can result in the mean absolute reduction in non-inflammatory lesions from 9 lesions to 20 lesions. In some embodiments, mean absolute reduction in non-inflammatory lesions after 3 weeks of treatment with a therapeutically effective amount of (4S,4aS,5aR,12aS)-4-dimethylamino-3,10,12,12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt may range from 1 lesion to 20 lesions. In some embodiments, mean absolute reduction in non-inflammatory lesions after 3 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt can range from 8 lesions to 12 lesions. Ins some embodiments, mean absolute reduction in non-inflammatory lesions after 3 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt can range from 9 lesions to 11 lesions. In some embodiments, mean absolute reduction in non-inflammatory lesions after 6 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt may range from 4 lesions to 24 lesions. In some embodiments, mean absolute reduction in non-inflammatory lesions after 6 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt can range from 12 lesions to 15 lesions. In some embodiments, mean absolute reduction in non-inflammatory lesions after 6 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt can range from 13 lesions to 14 lesions. In some embodiments, mean absolute reduction in non-inflammatory lesions after 9 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt may range from 6 lesions to 29 lesions. In some embodiments, mean absolute reduction in non-inflammatory lesions after 9 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt can range from 15 lesions to 20 lesions. In some embodiments, mean absolute reduction in non-inflammatory lesions after 9 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt can range from 16 lesions to 18 lesions. In some embodiments, mean absolute reduction in non-inflammatory lesions after 12 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt may range from 8 lesions to 29 lesions. In some embodiments, mean absolute reduction in non-inflammatory lesions after 12 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt can range from 17 lesions to 21 lesions. In some embodiments, mean absolute reduction in non-inflammatory lesions after 12 weeks of treatment with a therapeutically effective amount of (4S,4aS,5aR, 12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt can range from 18 lesions to 20 lesions.

The difference in mean absolute reduction in non-inflammatory lesions of a subject who receives a therapeutically effective amount of (4S,4aS,5aR,12aS)-4-dimethylamino-3,10,12,12a-tetrahydroxy-7-[(methoxy(methyl)amino)-methyl]-1,1 1-dioxo-1,4,4a,5,5a,6,11,12a-octahydro-naphthacene-2-carboxylic acid amide or a pharmaceutically acceptable salt compared to a placebo may range from 1 lesion to 15 lesions. In another embodiment, such administration may result in a difference in mean absolute reduction in non-inflammatory lesions from 1 lesion to 10 lesions. In yet another embodiment, such administration may result in a difference in mean absolute reduction in non-inflammatory lesions from 1 lesion to 8 lesions. In some embodiments, the difference in mean absolute reduction of non-inflammatory lesions after 3 weeks of treatment with a therapeutically effective amount of (4S,4aS,5aR,12aS)-4-dimethylamino-3, 10, 12, 12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt may range from 1 lesion to 7 lesions. In some embodiments, the difference in mean absolute reduction of non-inflammatory lesions after 3 weeks of treatment with a therapeutically effective amount of (4S,4aS,5aR,12aS)-4-dimethylamino-3,10,12,12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt can range from 1 lesion to 4 lesions. In some embodiments, the difference in mean absolute reduction of non-inflammatory lesions after 3 weeks of treatment with a therapeutically effective amount of (4S,4aS,5aR,12aS)-4-dimethylamino-3, 10, 12, 12a-tetrahydroxy-7-[(methoxy(methyl)amino)-methyl]-1,11-dioxo-1,4,4a, 5, 5 a,6,11,12a-octahydro-naphthacene-2-carboxylic acid amide or a pharmaceutically acceptable salt can range from 1 lesion to 2 lesions. In some embodiments, the difference in mean absolute reduction of non-inflammatory lesions after 6 weeks of treatment with a therapeutically effective amount of (4S,4aS,5aR,12aS)-4-dimethylamino-3,10,12,12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt may range from 1 lesion to 9 lesions. In some embodiments, the difference in mean absolute reduction of non-inflammatory lesions after 6 weeks of treatment with a therapeutically effective amount of (4S,4aS,5aR,12aS)-4-dimethylamino-3, 10, 12, 12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt can range from 1 lesion to 5 lesions. In some embodiments, the difference in mean absolute reduction of non-inflammatory lesions after 6 weeks of treatment with a therapeutically effective amount of (4S,4aS,5aR,12aS)-4-dimethylamino-3,10,12,12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt can range from 2 lesions to 3 lesions. In some embodiments, the difference in mean absolute reduction of non-inflammatory lesions after 9 weeks of treatment with a therapeutically effective amount of (4S,4aS,5aR,12aS)-4-dimethylamino-3, 10, 12, 12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt may range from 2 lesions to 11 lesions. In some embodiments, the difference in mean absolute reduction of non-inflammatory lesions after 9 weeks of treatment with a therapeutically effective amount of (4S,4aS,5aR,12aS)-4-dimethylamino-3,10,12,12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt can range from 1 lesion to 7 lesions. In some embodiments, the difference in mean absolute reduction of non-inflammatory lesions after 9 weeks of treatment with a therapeutically effective amount of (4S,4aS,5aR,12aS)-4-dimethylamino-3, 10, 12, 12a-tetrahydroxy-7-[(methoxy(methyl)amino)-methyl]-1,11-dioxo-1,4,4a, 5, 5 a,6,11,12a-octahydro-naphthacene-2-carboxylic acid amide or a pharmaceutically acceptable salt can range from 3 lesions to 5 lesions. In some embodiments, the difference in mean absolute reduction of non-inflammatory lesions after 12 weeks of treatment with a therapeutically effective amount of (4S,4aS,5aR,12aS)-4-dimethylamino-3,10,12,12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt may range from 1 lesion to 12 lesions. In some embodiments, the difference in mean absolute reduction of non-inflammatory lesions after 12 weeks of treatment with a therapeutically effective amount of (4S,4aS,5aR,12aS)-4-dimethylamino-3, 10, 12, 12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt can range from 1 lesion to 8 lesions. In some embodiments, the difference in mean absolute reduction of non-inflammatory lesions after 12 weeks of treatment with a therapeutically effective amount of (4S,4aS,5aR,12aS)-4-dimethylamino-3,10,12,12a-tetrahydroxy-7-[(methoxy(methyl)amino)-methyl]-1,11-dioxo-1,4,4a,5,5a,6,11,12a-octahydro-naphthacene-2-carboxylic acid amide or a pharmaceutically acceptable salt can range from 3 lesions to 6 lesions.

Unless defined otherwise, all technical and scientific terms used herein have the same meaning as commonly understood by one of ordinary skill in the art to which this invention belongs. The preferred methods and materials are now described..

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges which may independently be included in the smaller ranges also is encompassed within the invention, subject to any specifically excluded limit in the stated range. Where the stated range includes one or both of the limits, ranges excluding either both of those included limits also are included in the invention.

It must also be noted that as used herein and in the appended claims, the singular forms "a," "and" and "the" include plural referents unless the context clearly dictates otherwise. All technical and scientific terms used herein have the same meaning.

### EXAMPLES

The following examples are provided to further aid in understanding the embodiments disclosed in the application, and presuppose an understanding of conventional methods well known to those persons having ordinary skill in the art to which the examples pertain. The particular materials and conditions described hereunder are intended to exemplify particular aspects of embodiments disclosed herein.

### Example 1

Approximately 1.5 mg/kg per day of the *mono* hydrochloride crystalline salt of (4S,4aS,5aR,12aS)-4-dimethylamino-3, 10, 12, 12a-tetrahydroxy-7-[(methoxy(methyl)amino)-methyl]-1,11-dioxo-1,4,4a,5,5a,6,11,12a-octahydro-naphthacene-2-carboxylic acid amide (i.e., Formula I) was administered to 483 subjects, and a placebo was administered to 485 subjects. The subjects of this study included males and females 9 to 45 years of age with moderate to severe facial acne vulgaris and no disorders that would preclude the use of tetracycline-class antibiotics. Subjects were treated for a total of 12 weeks and returned to a clinic to be assessed on Weeks 3, 6, 9, and 12 of treatment. Subjects were assessed through lesion counts and Investigator's Global Assessment ("IGA") scores.

Subjects having facial acne vulgaris with: 20 to 50 inflammatory lesions (papules, pustules, and nodules), up to 100 non-inflammatory lesions (open and closed comedones), no more than two nodules and an IGA score of moderate (3) or severe (4) were included in the study.

The following is the IGA Scale used to assess acne vulgaris:

| **Score** | **Grade** | **Description** |
|---|---|---|
| 0 | Clear | No evidence of papules or pustules |
| 1 | Almost Clear | Rare: inflammatory papules (papules must be resolving and may be hyperpigmented, though not pink-red) |
| 2 | Mild | Few: inflammatory lesions (papules/pustules only; no nodulocytic lesions) |
| 3 | Moderate | Multiple: inflammatory lesions present; many papules/pustules; there may or may not be a few nodulocytic lesions |
| 4* | Severe | Inflammatory lesions are more apparent, many papules/pustules; there may or may not be a few nodulocytic lesions |

| | | |
|---|---|---|
| *Acne that worsens beyond Grade 4 must be recorded as an adverse event on the case report form (CRF). | | |

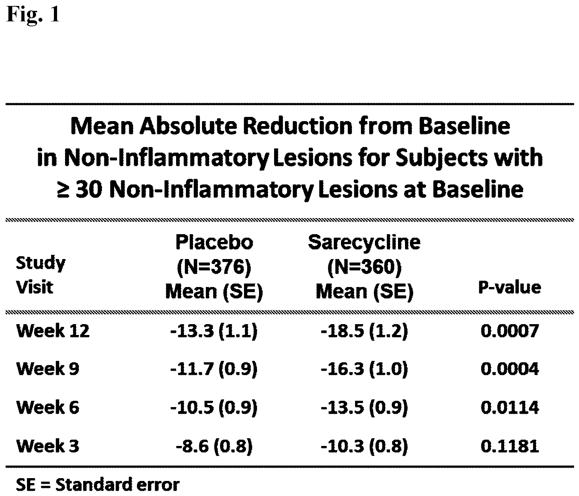

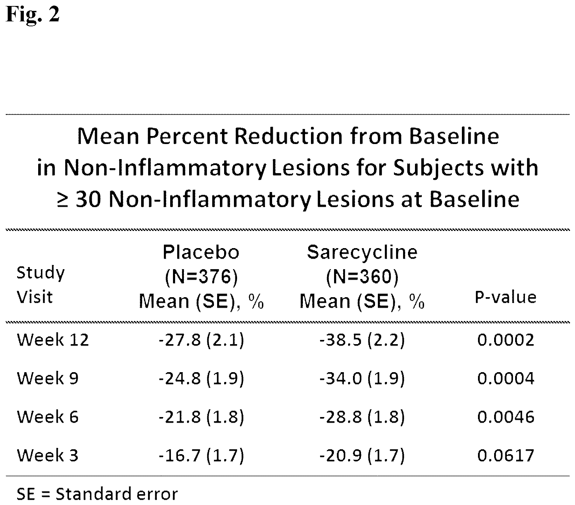

Data was analyzed for a subset of subjects having 30 or more non-inflammatory lesions. The effects of Formula I were evaluated for such subjects in comparison to a placebo. 360 subjects were given 1.5 mg/kg per day of Formula I, and 376 subjects were given a daily dose of a placebo. Each subject was instructed to take the daily dose at the same time each day at least 1 hour prior to or 2 hours after eating. Fig. 1 demonstrates the mean absolute reduction in non-inflammatory lesions for participants having 30 or more non-inflammatory lesions on the face at baseline who received daily doses of Formula I compared to the placebo. Fig. 2 demonstrates the mean percent reduction from baseline for subjects having 30 or more non-inflammatory lesions on the face at baseline. Results in these figures were based on a statistical (analysis of covariance) model used to estimate the mean treatment effects of Formula I and placebo, after adjusting for potential effects of investigative site and baseline value. In addition, the statistical superiority of Formula I relative to placebo is tested and p-values provided for each study visit.

### Example 2

Approximately 1.5 mg/kg per day of the *mono* hydrochloride crystalline salt of (4S,4aS,5aR,12aS)-4-dimethylamino-3, 10, 12, 12a-tetrahydroxy-7-[(methoxy(methyl)amino)-methyl]-1,11-dioxo-1,4,4a,5,5a,6,11,12a-octahydro-naphthacene-2-carboxylic acid amide (i.e., Formula I) was administered to 519 subjects, and a placebo was administered 515 subjects. The subjects of this study included males and females 9 to 45 years of age with moderate to severe facial acne vulgaris and no disorders that would preclude the use of tetracycline-class antibiotics. Subjects were treated for a total of 12 weeks and returned to a clinic to be assessed on Weeks 3, 6, 9, and 12 of treatment. Subjects were assessed through lesion counts and Investigator's Global Assessment ("IGA") scores (as described above in Example 1).

Subjects having facial acne vulgaris with: 20 to 50 inflammatory lesions (papules, pustules, and nodules), up to 100 non-inflammatory lesions (open and closed comedones), no more than two nodules and an IGA score of moderate (3) or severe (4) were included in the study.

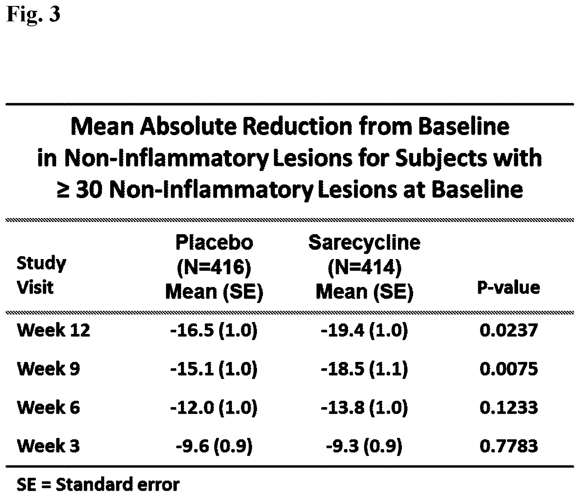

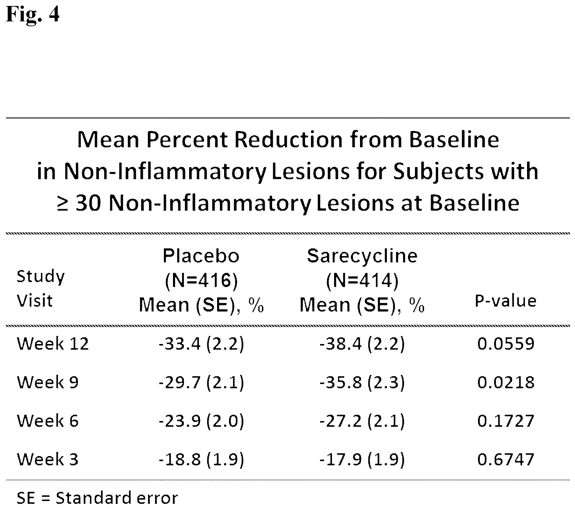

Data was analyzed for a subset of subjects having 30 or more non-inflammatory lesions. The effects of Formula I were evaluated for such subjects in comparison to a placebo. 414 subjects were given 1.5 mg/kg per day of Formula I, and 416 subjects were given a daily dose of a placebo. Each subject was instructed to take the daily dose at the same time each day. Fig. 3 demonstrates the mean absolute reduction in non-inflammatory lesions for participants having 30 or more non-inflammatory lesions on the face at baseline who received daily doses of Formula I compared to the placebo. Fig. 4 demonstrates the mean percent reduction from baseline for subjects having 30 or more non-inflammatory lesions on the face at baseline. Results in these figures were based on a statistical (analysis of covariance) model used to estimate the mean treatment effects of Formula I and placebo, after adjusting for potential effects of investigative site and baseline value. In addition, the statistical superiority of Formula I relative to placebo is tested and p-values provided for each study visit.

### Reference Example

Approximately 3.0 mg/kg per day, approximately 1.5 mg/kg per day, or approximately 0.75 mg/kg per day of the *mono* hydrochloride crystalline salt of (4S,4aS,5aR,12aS)-4-dimethylamino-3, 10, 12, 12a-tetrahydroxy-7-[(methoxy(methyl)amino)-methyl]-1,11-dioxo-1,4,4a,5,5a,6,11,12a-octahydro-naphthacene-2-carboxylic acid amide (i.e., Formula I) compared to a placebo was administered to subjects including approximately 280 male and female subjects aged 12 to 45 years having moderate to severe facial acne vulgaris. Subjects were randomly assigned in a 1:1:1:1 ratio to one of the said four treatment groups. Subjects were treated for a total of 12 weeks and returned to a clinic to be assessed on Weeks 1, 2, 4, 8, and 12 of treatment. Subjects were assessed through lesion counts and Investigator's Global Assessment ("IGA") scores.

Subjects having facial acne vulgaris with: 20 to 50 inflammatory lesions, 30 to 100 non-inflammatory lesions, no more than 2 facial nodules and having an Investigator Global Assessment (IGA) at Baseline Visit of "moderate" or "severe" were included in the study.

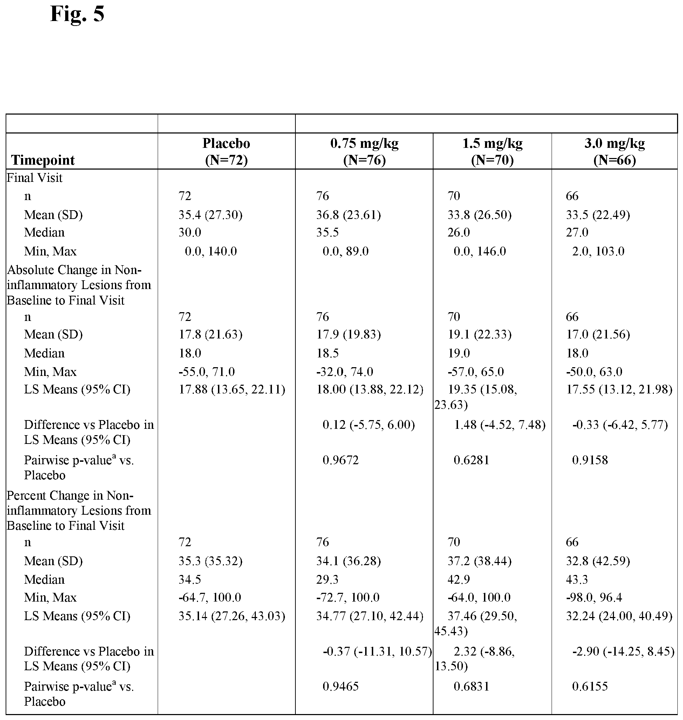

The effects of Formula I were evaluated in comparison to a placebo. In this study, 72 subjects were given a placebo, 76 subjects were given 0.75 mg/kg per day of Formula I, 70 subjects were given 1.5 mg/kg per day of Formula I and 66 subjects were given 3.0 mg/kg per day of Formula I. Each subject was instructed to take the daily dose at the same time each day. Fig. 5 demonstrates the mean absolute and percent change from baseline to final visit.

## Claims

1. A compound, which compound is (4S,4aS,5aR,12aS)-4-dimethylamino-3,10,12,12a-tetrahydroxy-7- [(methoxy(methyl)amino)-methyl]-1,11-dioxo-1,4,4a,5,5a,6,11,12a-octahydro- naphthacene-2-carboxylic acid amide or a pharmaceutically acceptable salt thereof, for use in a therapeutically effective amount in the treatment of non-inflammatory acne vulgaris lesion.

2. The compound for use according to claim 1, wherein the pharmaceutically acceptable salt is selected from the group consisting of a crystalline *mono* hydrochloride, a crystalline *mono* mesylate, and a crystalline *mono* sulfate salt.

3. The compound for use according to claim 1, wherein the pharmaceutically acceptable salt is crystalline *mono* hydrochloride.

4. The compound for use according to any one of the preceding claims, wherein the therapeutically effective amount is administered daily.

5. The compound for use according to any one of the preceding claims, wherein the therapeutically effective amount is administered for at least three weeks, at least six weeks, at least nine weeks, or at least twelve weeks.

6. The compound for use according to any one of the preceding claims, wherein the therapeutically effective amount is 1.1 mg/kg/day to 1.8 mg/kg/day.

7. The compound for use according to any one of the preceding claims, wherein the therapeutically effective amount is 1.5 mg/kg/day.

8. The compound for use according to any one of the preceding claims, wherein the therapeutically effective amount is administered as an oral dosage form.

9. The compound for use according to any one of the preceding claims wherein the non-inflammatory acne vulgaris lesion is facial acne vulgaris.

10. The compound for use according to any one of claims 1 to 9, wherein the non-inflammatory acne vulgaris lesion is an open comedone.

11. The compound for use according to any one of claims 1 to 9, wherein the non-inflammatory acne vulgaris lesion is a closed comedone.

## Patentansprüche

1. Verbindung, wobei diese Verbindung (4S,4aS,5aR,12aS)-4-Dimethylamino-3,10,12,12a-tetrahydroxy-7-[(methoxy(methyl)amino)-methyl]-1,11-dioxo-1,4,4a,5,5a,6,11,12a-octahydro-naphthacen-2-carbonsäureamid oder ein pharmazeutisch unbedenkliches Salz davon für die Verwendung in einer therapeutisch wirksamen Menge bei der Behandlung von nicht-entzündlichen Acne vulgaris-Läsionen ist.

2. Verbindung für die Verwendung nach Anspruch 1, wobei das pharmazeutisch unbedenkliche Salz aus der Gruppe ausgewählt ist, bestehend aus einem kristallinen *Mono*hydrochlorid, einem kristallinen *Mono*mesylat und einem kristallinen *Mono*sulfatsalz.

3. Verbindung für die Verwendung nach Anspruch 1, wobei das pharmazeutisch unbedenkliche Salz kristallines *Mono*hydrochlorid ist.

4. Verbindung für die Verwendung nach einem der vorhergehenden Ansprüche, wobei die therapeutisch wirksame Menge täglich verabreicht wird.

5. Verbindung für die Verwendung nach einem der vorhergehenden Ansprüche, wobei die therapeutisch wirksame Menge für wenigstens drei Wochen, wenigstens sechs Wochen, wenigstens neun Wochen oder wenigstens zwölf Wochen verabreicht wird.

6. Verbindung für die Verwendung nach einem der vorhergehenden Ansprüche, wobei die therapeutisch wirksame Menge 1,1 mg/kg/Tag bis 1,8 mg/kg/Tag beträgt.

7. Verbindung für die Verwendung nach einem der vorhergehenden Ansprüche, wobei die therapeutisch wirksame Menge 1,5 mg/kg/Tag beträgt.

8. Verbindung für die Verwendung nach einem der vorhergehenden Ansprüche, wobei die therapeutisch wirksame Menge als eine orale Dosierungsform verabreicht wird.

9. Verbindung für die Verwendung nach einem der vorhergehenden Ansprüche, wobei die nicht-entzündliche Acne vulgaris-Läsion Acne vulgaris im Gesicht ist.

10. Verbindung für die Verwendung nach einem der Ansprüche 1 bis 9, wobei die nicht-entzündliche Acne vulgaris-Läsion ein offenes Komedo ist.

11. Verbindung für die Verwendung nach einem der Ansprüche 1 bis 9, wobei die nicht-entzündliche Acne vulgaris-Läsion ein geschlossenes Komedo ist.

## Revendications

1. Composé, lequel composé est l'amide d'acide (4S,4aS,5aR,12aS)-4-diméthylamino-3,10,12,12a-tétrahydroxy-7-[(méthoxy(méthyl)amino)-méthyl]-1,11-dioxo-1,4,4a,5,5a,6,11,12a-octahydro-naphtacène-2-carboxylique ou un sel pharmaceutiquement acceptable de celui-ci, pour une utilisation en une quantité thérapeutiquement efficace dans le traitement d'une lésion d'acné vulgaire non inflammatoire.

2. Composé pour une utilisation selon la revendication 1, dans lequel le sel pharmaceutiquement acceptable est choisi dans le groupe constitué par un sel *mono* chlorhydrate cristallin, un sel *mono* mésylate cristallin, et un sel *mono* sulfate cristallin.

3. Composé pour une utilisation selon la revendication 1, dans lequel le sel pharmaceutiquement acceptable est un sel *mono* chlorhydrate cristallin.

4. Composé pour une utilisation selon l'une quelconque des revendications précédentes, dans lequel la quantité thérapeutiquement efficace est administrée quotidiennement.

5. Composé pour une utilisation selon l'une quelconque des revendications précédentes, dans lequel la quantité thérapeutiquement efficace est administrée pendant au moins trois semaines, au moins six semaines, au moins neuf semaines, ou au moins douze semaines.

6. Composé pour une utilisation selon l'une quelconque des revendications précédentes, dans lequel la quantité thérapeutiquement efficace est de 1,1 mg/kg/jour à 1,8 mg/kg/jour.

7. Composé pour une utilisation selon l'une quelconque des revendications précédentes, dans lequel la quantité thérapeutiquement efficace est de 1,5 mg/kg/jour.

8. Composé pour une utilisation selon l'une quelconque des revendications précédentes, dans lequel la quantité thérapeutiquement efficace est administrée sous forme posologique orale.

9. Composé pour une utilisation selon l'une quelconque des revendications précédentes dans lequel l'acné vulgaire non inflammatoire est l'acné vulgaire faciale.

10. Composé pour une utilisation selon l'une quelconque des revendications 1 à 9, dans lequel la lésion d'acné vulgaire non inflammatoire est un comédon ouvert.

11. Composé pour une utilisation selon l'une quelconque des revendications 1 à 9, dans lequel la lésion d'acné vulgaire non inflammatoire est un comédon fermé.
